# DEMANDE DE BREVET EUROPEEN

(11) **EP 0 668 068 A1**
(43) Date de publication de la demande: **23.08.1995**
(21) Numéro de dépôt: 95400098.0
(22) Date de dépôt: 18.01.1995
(51) Int. Cl.: A61J 3/07

(54) **Médicament ayant une présentation pédiatrique facilitant son absorption par un enfant**

(30) Priorité: 21.01.1994 FR 9400639
(71) Demandeur: Grimberg, Georges Serge, F-75007 Paris (FR)
(72) Inventeur: Grimberg, Georges Serge, F-75007 Paris (FR)
(74) Mandataire: Madeuf, René Louis

(57) **Abrégé**

Le médicament se présente sous la forme d'une gélule qui est constituée par un petit récipient à fond creux en cuvette, relié par une bande souple à un chapeau de forme correspondante appropriée avec un clapet de maintien du chapeau sur la cuvette facile à manoeuvrer pour ouvrir la gélule contenant la dose médicamenteuse.

## Description

Donner un médicament à un jeune enfant demande l'acceptation de celui-ci, d'où la nécessité d'édulcorer et d'aromatiser un médicament. La forme liquide est la forme la plus facile à préparer et à administrer. Cependant, celle-ci comporte deux inconvénients :
- il est indispensable de mettre des conservateurs en quantités relativement importantes par rapport au poids de l'enfant.
- un liquide doit être pris à la cuiller. La précision n'est jamais parfaite et le médicament peut être pris en dehors d'une alimentation, donc être dans un estomac vide.

Si le produit est, par exemple, un sel de magnésium, il doit être pris mélangé à de l'alimentation car, chez certains, il provoque de la diarrhée et des douleurs stomacales lorsqu'il est pris en dehors des repas.

L'objet de la présente invention est de fournir une poudre mélangeable à de l'alimentation en quantité parfaitement dosée, donc une poudre en dose unitaire et sous un petit volume. Il est précisé "sous un petit volume" car la forme sachet existe mais cela représente obligatoirement un volume relativement important, fait notamment d'excipients qui, en général, ne sont pas adaptés à la nourriture d'un bébé.

De plus, il existe des doses unitaires dites formes sèches qui s'avalent, par exemple des comprimés ou des gélules. Dans certains cas, ces formes sont dangereuses à avaler, peuvent provoquer des problèmes stomacaux ou être éliminées sans avoir été absorbées.

On connaît par US-A-4 076 848 des gélules contenant un mélange de légumes et d'épices déshydratés réduits en poudre, et destinés à aromatiser différentes sortes de préparations culinaires. Ces gélules ont donc été conçues pour pouvoir être facilement ouvertes puis refermées de manière à choisir la quantité de produit voulu.

La présente invention a pour objet une gélule de type totalement différent. En effet, cette gélule s'ouvre facilement permettant ainsi à la mère de l'enfant de verser le contenu de cette gélule, par exemple un sel de magnésium auquel est ajouté un édulcorant ou un arôme, le tout sous la forme de poudre dans un aliment très facilement accepté par un enfant en bas âge, par exemple un yaourt, une crème sucrée ou un produit analogue.

On voit ainsi que le but de la présente invention est totalement différent de celui mentionné dans US-A-4 076 848 ci-dessus. Dans la présente invention, il s'agit en effet de la prise d'un médicament qui se présente sous la forme d'une poudre en dose unitaire. Ainsi, chaque gélule contient une certaine quantité de poudre parfaitement dosée et doit s'ouvrir facilement. En aucun cas, cette gélule a été conçue pour pouvoir être refermée étant donné qu'à chaque fois la gélule est vidée intégralement.

Conformément à l'invention, la gélule est constituée par un petit récipient à fond creux en cuvette, relié par une bande souple à un chapeau de forme correspondante appropriée avec un clapet de maintien du chapeau sur la cuvette facile à manoeuvrer pour ouvrir la gélule contenant la dose médicamenteuse.

Suivant une autre caractéristique de l'invention, la dose médicamenteuse se présente sous la forme d'une poudre très fine anhydre le plus souvent facilement mélangeable à un aliment liquide, semi-liquide ou pâteux qui est facilement ingéré par un enfant en bas âge.

Suivant une autre particularité de l'invention, le médicament contient :

| | |
|---|---|
| Sulfate de magnésium 3H₂O | 355 mg |
| Levure saccharomyces | 50 mg |
| Saccharinate de sodium | 5 mg |
| Cyclamate de sodium | 10 mg |
| Acide citrique | 10 mg |
| Arôme d'orange | 10 mg |
| Aérosil | 1 mg |

pour une gélule.

Diverses autres caractéristiques de l'invention ressortent d'ailleurs de la description détaillée qui suit.

A titre d'exemple, on utilise dans le médicament, objet de la présente invention, une gélule qui se compose d'une partie inférieure en forme de cuvette reliée au chapeau de la gélule par une bande souple formant charnière, la cuvette et le chapeau de forme correspondant à la cuvette pouvant présenter, par exemple dans la zone opposée à la charnière, un petit redan formant clapet pour maintenir fermée la gélule lorsque celle-ci a été remplie avec le médicament. Comme il apparaît donc, il est facile par une simple pression sur la cuvette de faire échapper le redan du bord inférieur du chapeau qui est également le plus souvent en forme de petite cuvette afin d'ouvrir la gélule et de pouvoir la vider aisément, sans qu'il soit possible de la refermer.

En général, la gélule a, en section, une forme circulaire, la cuvette et le chapeau étant légèrement bombés mais cette forme pouvant être aisément remplacée par une forme autre telle qu'une forme carrée permettant ainsi, comme dans le cas de la forme ronde, un empilement aisé dans le cartonnage d'emballage du médicament.

Chaque gélule est remplie en général d'un médicament à base de sel de magnésium, médicament dont la composition est la suivante :

| | |
|---|---|
| Sulfate de magnésium 3H₂O | 355 mg |
| Levure saccharomyces | 50 mg |
| Saccharinate de sodium | 5 mg |
| Cyclamate de sodium | 10 mg |
| Acide citrique | 10 mg |
| Arôme d'orange | 10 mg |
| Aérosil | 1 mg |

pour une gélule.

Bien entendu et dans le même genre de médicament, à la place du sel de magnésium servant de principe actif dans le cas cité ci-dessus, on peut utiliser d'autres substances thérapeutiques actives.

A titre de test, dix enfants, devant être traités par un sel de magnésium sur une période de plusieurs semaines, ont été divisés en deux groupes. Cinq des enfants ont reçu une composition à base de sel de magnésium à laquelle étaient ajoutés de la levure saccharomyces, du saccharinate de sodium, du cyclamate de sodium et un excipient.

Le premier groupe d'enfants a utilisé des ampoules buvables contenant du sulfate de magnésium.

Le deuxième groupe d'enfants a été traité à l'aide du médicament décrit dans l'exemple cité ci-dessus et présenté sous la forme de la gélule sus-décrite.

Il a été observé pour le premier groupe d'enfants des réactions secondaires, en particulier des douleurs stomacales et dans un cas une diahrrée difficilement résorbée.

Pour le deuxième groupe d'enfants, aucune réaction secondaire n'a été constatée, la tolérance semblant être parfaite et en tout cas notoirement meilleure.

## Revendications

**1 -** Médicament ayant une présentation pédiatrique facilitant son absorption par un enfant, caractérisé en ce qu'une gélule est constituée par un petit récipient à fond creux en cuvette, relié par une bande souple à un chapeau de forme correspondante appropriée avec un clapet de maintien du chapeau sur la cuvette facile à manoeuvrer pour ouvrir la gélule contenant la dose médicamenteuse.

**2 -** Médicament suivant la revendication 1, caractérisé en ce que la dose médicamenteuse se présente sous la forme d'une poudre très fine anhydre le plus souvent facilement mélangeable à un aliment liquide, semi-liquide ou pâteux qui est facilement ingéré par un enfant en bas âge.

**3 -** Médicament suivant les revendications 1 et 2, caractérisé en ce que le médicament contient :
| | |
|---|---|
| Sulfate de magnésium 3H₂O | 355 mg |
| Levure saccharomyces | 50 mg |
| Saccharinate de sodium | 5 mg |
| Cyclamate de sodium | 10 mg |
| Acide citrique | 10 mg |
| Arôme d'orange | 10 mg |
| Aérosil | 1 mg |
pour une gélule.

**4 -** Médicament suivant les revendications 1 à 3, caractérisé en ce que le principe actif peut être toutes autres substances thérapeutiquement actives.

**5 -** Médicament ayant une présentation pédiatrique facilitant son absorption par un enfant sensiblement tel que décrit.
